# EUROPÄISCHE PATENTSCHRIFT

(11) **EP 3 586 526 B1**
(45) Veröffentlichungstag und Bekanntmachung des Hinweises auf die Patenterteilung: **13.04.2022**
(21) Anmeldenummer: 18714959.6
(22) Anmeldetag: 23.02.2018
(51) Int. Cl.: H04R 25/00, A61B 5/12

(54) **VERFAHREN ZUR PATIENTENSPEZIFISCHEN KONFIGURATION EINES HÖRGERÄTES UND VERFAHREN ZUM ERZEUGEN VON TESTSIGNALEN DAFÜR**
METHOD FOR PROVIDING A CUSTOMIZABLE CONFIGURATION OF A HEARING AID AND METHOD FOR GENERATING TEST SIGNALS THEREFORE
PROCÉDÉ POUR CONFIGURER UNE PROTHÈSE AUDITIVE PERSONNALISABLE ET SON PROCÉDÉ DE GÉNÉRATION DES SIGNAUX DE TEST

(30) Priorität: 23.02.2017 DE 102017103808
(43) Veröffentlichungstag der Anmeldung: 01.01.2020
(73) Patentinhaber: Hörwelt Duisburg GmbH, 47057 Duisburg (DE)
(72) Erfinder: BECKER, Kristof, 47198 Duisburg (DE); MÜLLER, Thomas Stefan, 47229 Duisburg (DE)
(74) Vertreter: CBDL Patentanwälte
(86) Internationale Anmeldenummer: PCT/DE2018/100164
(87) Internationale Veröffentlichungsnummer: WO 2018/153412

(56) Entgegenhaltungen:
- EP-A2- 1 744 590
- EP-B1- 1 744 590
- DE-A1- 2 843 923
- DE-A1- 2 843 923
- DE-A1- 4 338 215
- DE-A1- 4 339 898
- DE-A1-102009 032 238
- DE-C2- 4 338 215
- DE-U1-202016 101 050

## Beschreibung

### TECHNISCHES GEBIET DER ERFINDUNG

Die Erfindung betrifft ein Verfahren zur patientenspezifischen Konfiguration eines Hörgerätes und ein Verfahren zum Erzeugen von Testsignalen dafür.

### HINTERGRUND DER ERFINDUNG

Hörbeeinträchtigungen betreffen in der Regel nur bestimmte Frequenzen und bestimmte Lautstärken, d.h. Patienten mit einer Hörbeeinträchtigung können Tonsignale bestimmter Frequenzen wie Personen mit normalem Gehör hören, anderer Frequenzen dagegen nicht, wobei zudem oft auch die Eingangslautstärke eine Rolle spielt. Dabei sei an dieser Stelle darauf hingewiesen, das unter dem Begriff Tonsignale hier alle Arten von Schallereignissen verstanden werden, die das menschliche Trommelfell im normalerweise hörbaren Frequenz- und Laustärkebereich zu Schwingungen anregen, also sowohl reine Sinustöne, Musik, alle Arten von Rauschen und Geräuschen, Sprache etc. Auch die nachfolgend genannten Testsignale sind in diesem Sinne Tonsignale.

Moderne Hörgeräte ermöglichen es, unterschiedlichste Hörbeeinträchtigungen dadurch auszugleichen, dass sie eingehende Tonsignale in eine Anzahl, z.B. vier oder mehr, verschiedener Frequenzbänder unterteilen und in jedem Frequenzband die Tonsignale in Abhängigkeit von der jeweiligen Eingangslautstärke mit unterschiedlichen Verstärkungsfaktoren, die individuell konfigurierbar sind, verstärken. Üblich sind drei sog. Dynamikstufen, nämlich eine untere Dynamikstufe mit pro Frequenzband jeweils einem ersten Verstärkungsfaktor für niedrige Eingangslaustärkepegel bis z.B. 50 dB, eine mittlere Dynamikstufe mit pro Frequenzband jeweils einem zweiten Verstärkungsfaktor für mittlere Eingangslaustärkepegel zwischen z.B. 50 und 80 dB und eine obere Dynamikstufe mit pro Frequenzband jeweils einem dritten Verstärkungsfaktor für hohe Eingangslaustärkepegel über 80 dB. Die frequenzbbandspezifischen Verstärkungsfaktoren bilden die sog. Verstärkungsmatrix und können über eine Anpasssoftware patientenspezifisch konfiguriert werden. Eben dieses Konfigurieren ist Gegenstand der Erfindung.

Sowohl das Bestimmen der sog. patientenspezifischen Hörschwelle, d.h. des Verlaufs der Kurve Frequenz zu gerade noch hörbarer Lautstärke, als auch die sich daraus theoretisch ergebende Konfiguration eines Hörgeräts sind seit langem bekannt. Allerdings hat sich in der Praxis gezeigt, dass die aus der Hörschwelle berechnete theoretische Konfiguration eines Hörgerätes praktisch nie zu einem befriedigenden Hörerlebnis für den Patienten führt und allenfalls als Grundkonfiguration für eine aufwendige individuelle Konfiguration der verschiedenen Verstärkungsfaktoren dienen kann, denn das menschliche Hören ist ein überaus komplexer Vorgang, bei dem verschiedenste Faktoren eine Rolle spielen. So ist jedes Ohr individuell geformt und Form und Volumen des Gehörgangs, Steifigkeit des Trommelfells, Gehörgangresonanzen etc. spielen bei der Wahrnehmung eine Rolle. Handelt es sich bei dem Hörgerät um eine Versorgung mit maßgefertigter Otoplastik, also mit einem für den Gehörgang des Kunden individuell gefertigten Ohrpassstück, beeinflusst das Hörgerät selbst die Hörwahrnehmung, da es eintreffenden Schall, selbst wenn dieser von an sich wahrnehmbarer Lautstärke und Frequenz ist, zwangsläufig dämpft. Dieser Effekt tritt auch, wenngleich in geringerem Maße, bei Hinterohrgeräten auf, bei denen nur ein dünner Schallschlauch oder ein ausgelagerter Hörer mit entsprechender Ankopplung in den Gehörgang gesetzt ist.

Die Konfiguration eines Hörgerätes, insbesondere die exakte Konfiguration der einzelnen Verstärkungsfaktoren, erweist sich in der Praxis als überaus zeitaufwändiger und für den Patienten äußerst unbefriedigender Vorgang, der es regelmäßig notwendig macht, dass Patienten mehrfach Nachkonfigurationen bei einem Hörgeräteakustiker vornehmen lassen müssen und nicht selten aufgrund des unbefriedigenden Hörerlebnisses das Tragen des Hörgerätes ganz einstellen.

Um die Konfiguration eines Hörgerätes zu erleichtern, wurden bereits verschiedene Verfahren vorgeschlagen. So lehrt die EP 1 746 859 B1 ein Verfahren, bei dem einem ein Hörgerät tragenden Patienten über einen sogenannten offenen Kopfhörer mittels eines an sich bekannten Audiometers Testsignale in einer für Normalhörende hörbaren festen Lautstärke vorgespielt werden und die frequenzbandabhängige Verstärkung am Hörgerät bis zum Erreichen der Hörschwelle des Patienten eingestellt wird. Das Hörgerät übernimmt dabei durch Verstärken der Testsignale quasi einen Teil der üblichen Funktion eines Audiometers, mittels welchem an sich die Lautstärke der Töne verändert wird, um die Hörschwelle zu bestimmen. Der Patient gibt dem die Konfiguration vornehmenden Hörgeräteakustiker fortwährend Rückmeldung darüber, wann er das jeweilige Testsignal hört. Dabei sei an dieser Stelle darauf hingewiesen, dass im nachfolgenden zwar der Einfachheit halber immer nur von einem ein Hörgerät tragenden Patienten gesprochen wird, der Patient typischerweise aber zwei Hörgeräte, nämlich in jedem Ohr eines, trägt, wobei dies natürlich von der patientenspezifischen Hörbeeinträchtigung abhängt. Es spielt aber für das Verfahren zur Konfiguration eines Hörgerätes keine Rolle, ob der Patient ein oder zwei Hörgeräte benötigt.

Es hat sich gezeigt, dass die Konfiguration eines Hörgeräts durch eine frequenzbandspezifische Verstärkung jeweils bis zum Erreichen der Hörschwelle des Patienten nicht zu befriedigenden Ergebnissen führt, was unter anderem daran liegt, dass es während der Konfiguration für den Patienten schwierig ist, das Auftreten eines verstärkten Signals an der Hörschwelle präzise zu identifizieren.

Um beim Konfigurieren eines Hörgerätes tatsächliche Hörsituationen zu simulieren, schlägt die DE 10 2007 054 152 A1 die Verwendung eines Lautsprecherarrays vor, mittels welchem durch Wellenfeldsynthese virtuelle Tonquellen in einem Testraum, in dem sich der Patient befindet, simuliert werden können und der Patient dann Rückmeldung über die empfundenen Schalleindrücke gibt. Da sich der Patient dabei aber mehr oder weniger frei bewegen kann, verfälschen bereits leichte Kopfbewegungen das Ergebnis, so dass sich auch dieses Verfahren in der Praxis nicht durchgesetzt hat.

Die DE 10 2011 104 536 A1 beschreibt ein Verfahren, bei dem ein ein Hörgerät tragender Patient in einem freien Schallfeld mit festen Signalen ansteigender Lautstärke beschallt wird, bis er zu erkennen gibt, Signale wahrgenommen zu haben. Das Hörgerät wird anhand dieser Erkenntnisse voreingestellt. Anschließend werden am Hörgerät Schwellenwerte für die Hörschwelle, einen sogenannten Hörbereich und einen sogenannten Unbehaglichkeitsbereich eingestellt.

Die DE 20 2016 101 050 U1, die derzeit als nächstkommender Stand der Technik angesehen wird, beschreibt ein Verfahren zum Konfigurieren eines Hörgerätes, bei dem ein das Hörgerät tragender Patient ebenfalls einem freien Schallfeld ausgesetzt und dem Patienten Testsignale verschiedener Frequenzen aber gleicher Lautstärke vorgespielt werden, worauf der Patient bewertet, ob er die beiden physikalisch tatsächlich gleich lauten Signale auch gleich laut empfunden hat. Als Testsignale werden dabei Sinussignale, Terzen oder schmalbandiges Rauschen verwendet, die dem Patienten mit sogenannter "deutlich überschwelliger" Lautstärke, d.h. einer Lautstärke, die für Normalhörende deutlich hörbar ist, typischerweise mit einem Pegel von 45 bis 80 dB vorgespielt werden. Bevorzugt werden die Testsignale mit 65 dB vorgespielt, was etwas oberhalb der Lautstärke einer normalen Unterhaltung liegt. Allerdings führt die Verwendung deutlich überschwelliger Testsignale bei der Konfiguration eines Hörgerätes dazu, dass leise Sprachanteile, wie insbesondere die Konsonanten s, t und f, die in der deutschen Sprache entscheidend zur Wortdifferenzierung beitragen, nicht hinreichend verstärkt werden.

Ein besonderes Problem bei der Konfiguration von Hörgeräten stellt die Tatsache dar, dass objektiv gleichlaute Tonsignale, also Tonsignale, die denselben messbaren Schalldruck erzeugen, vom Menschen unterschiedlich laut wahrgenommen werden. Hohe Töne werden gemeinhin lauter empfunden als physikalische gleichlaute tiefe Töne, was einerseits damit zu tun hat, dass hohe Töne schneller einschwingen und signaltechnisch schneller verarbeitet werden können, andererseits auch entwicklungsgeschichtlich begründbar ist, weil höhere Tonsignale, wie zum Beispiel Knacken von Holz tendenziell eher potentielle Gefahren signalisierten als tiefere Tonsignale. Dies führt bei einer Konfiguration eines Hörgerätes gemäß der Lehre der DE 10 2016 101 050 U1 dazu, dass hohe Frequenzbereiche typischerweise zu wenig verstärkt werden, da der Patient die hochfrequenten Anteile der dort verwendeten Testsignale, wie zum Beispiel einem sogenannten rosa Rauschen, als lauter im Vergleich zu dessen niederfrequenten Anteilen empfindet.

Aus der DE 10 2009 032 238 A1 ist ein Verfahren zur Überprüfung der bei der Anpassung eines Hörgerätes vorgenommenen Einstellungen bekannt, wobei die Sprachverständlichkeit nach bereits erfolgter Hörgeräteanpassung, insbesondere die Wahrnehmbarkeit einzelner Phoneme getestet wird. Dabei wird allerdings die Lautheit im allgemeinen Sinn nicht bewertet, sondern lediglich die Hörbarkeit schwellennaher Phoneme geprüft, unabhängig davon, ob die Hörgeräteeinstellungen evtl. zu unnatürlich lauten und oder gar belastenden Verstärkungen führen.

Die DE 43 38 215 A1 schlägt die Verwendung von natürlichen Klangmustern wie z.B. Musik und Alltagsgeräuschen zur Ersteinstellung eines Hörgerätes vor, insbesondere von Musik und Geräuschen, die dem jeweiligen Patienten bereits bekannt sind. Allerdings hat sich dieser Verfahren in der Praxis nicht durchgesetzt, da sich gezeigt hat, dass das Wiedererkennen von bekannten Klangmustern gerade dazu führt, dass ihre Lautheit völlig falsch eingeschätzt wird. Ähnlich wie bei einem Sehtest, wo der Patient ein bekanntes Muster, z.B. ein Wort, erkennt ohne tatsächlich die einzelnen Buchstaben genau lesen zu können, führt die Verwendung natürlicher Klangmuster zu Wiedererkennungseffekten ohne dass der Patient die einzelnen Töne genau gehört hat.

### OFFENBARUNG DER ERFINDUNG

Der Erfindung liegt die Aufgabe zugrunde, ein Verfahren zum Konfigurieren eines Hörgeräts anzugeben, das es ermöglicht, in einfacher Weise ein Hörgerät so für einen Patienten zu konfigurieren, dass es zu einem möglichst natürlichen Hörerlebnis führt. Dabei soll in einer bevorzugten Ausführungsform gewährleistet sein, dass auch die leisen Sprachanteile, insbesondere die oben genannten Konsonanten, ausreichend verstärkt werden. Da die Erfindung überraschend erkannt hat, dass ein gemeinsames Problem aller bislang bekannten Verfahren zur Konfiguration von Hörgeräten in der Verwendung bestimmter Testsignale liegt, ist es eine weitere Aufgabe der Erfindung, ein Verfahren zum Erzeugen eines Testsignals anzugeben, das bei der Konfiguration von Hörgeräten vorteilhaft verwendet werden kann.

Die Aufgabe wird gelöst von einem Verfahren zur Konfiguration eines Hörgerätes mit den Merkmalen des Anspruchs 1 beziehungsweise von einem Verfahren zum Erzeugen eines Testsignals mit den Merkmalen des Anspruchs 11. Der nebengeordnete Anspruch 13 betrifft die Verwendung eines erfindungsgemäß erzeugten Testsignals zum Konfigurieren eines Hörgeräts. Vorteilhafte Ausgestaltungen und Weiterbildungen der Erfindung sind Gegenstand der Unteransprüche.

Die Erfindung beruht auf der überraschenden Erkenntnis, dass die während der Konfiguration eines Hörgerätes dem Patienten vorgespielten Testsignale ganz massiven Einfluss auf die Qualität der Konfiguration, d.h. auf das Hörempfinden, das der Patient beim Tragen des Hörgerätes im Alltag erlebt, haben. Durch Anwendung jüngerer Erkenntnisse der Psychoakustik, insbesondere hinsichtlich der Lautstärkewahrnehmung von Tonsignalen objektiv gleicher Lautstärke, lässt sich ein Hörgerät vorteilhaft in typischerweise einer einzigen Konfigurationssitzung so konfigurieren, dass der Patient damit nach kurzer Eingewöhnungsphase, innerhalb derer er sich wie bei anderen Hilfsmitteln, zum Beispiel Brillen, wieder an die natürliche Wahrnehmung von Umwelteindrücken gewöhnen muss, ein ausgezeichnetes Hörempfinden erlebt. Dies hat auch enorme wirtschaftliche Bedeutung, da bislang regelmäßig mehrere Konfigurationssitzungen mit entsprechendem Zeitaufwand sowohl für den Patienten als auch für den Hörgeräteakustiker notwendig waren, um, wenn überhaupt, ein akzeptables Hörerlebnis zu erzielen.

Weitere Einzelheiten und Vorteile der Erfindung ergeben sich aus der nachfolgenden rein beispielhaften und nicht-beschränkenden Beschreibung bevorzugter Ausführungs- und Durchführungsformen in Verbindung mit der sechs Figuren umfassenden Zeichnung.

### KURZE BESCHREIBUNG DER ZEICHNUNG

- Fig. 1: zeigt ein dreidimensionales Spektrogramm eines erfindungsgemäßen Testsignals aus einer ersten Perspektive.
- Fig. 2: zeigt das Spektrogramm gemäß Fig. 1 aus einer zweiten Perspektive.
- Fig. 3: zeigt das Spektrogramm gemäß Fig. 1 aus einer dritten Perspektive.
- Fig. 4: zeigt ein dreidimensionales Spektrogramm eines bislang typischerweise verwendeten Testsignals.
- Fig. 5: zeigt das Spektrogramm gemäß Fig. 4 aus einer zweiten Perspektive.
- Fig. 6: zeigt das Spektrogramm gemäß Fig. 4 aus einer dritten Perspektive.

### BESCHREIBUNG BEVORZUGTER AUSFÜHRUNGSFORMEN

Erfindungsgemäß wird ein Hörgerät, das eingehende Tonsignale in eine Anzahl verschiedener Frequenzbänder unterteilt und eingangslautstärkenabhängig verstärkt, wobei pro Frequenzband wenigstens drei Verstärkungsfaktoren individuell konfigurierbar sind, nämlich ein erster Verstärkungsfaktor für niedrige Eingangslaustärkepegel (typischerweise bis etwa 50 dB), ein zweiter Verstärkungsfaktor für mittlere Eingangslaustärkepegel (typischerweise von etwa 50 bis 80 dB) und ein dritter Verstärkungsfaktor für hohe Eingangslaustärkepegel (ab etwa 80 dB) dadurch patientenspezifisch konfiguriert, das einem das Hörgerät tragenden Patienten Testsignale, die jeweils ein anderes Frequenzband umfassen, mit demselben Eingangslautstärkepegel vorgespielt werden und der Patient angibt, ob er die Testsignale gleichlaut hört, und, wenn nicht, der jeweilige Verstärkungsfaktor angepasst wird, wobei die Testsignale auf zusammengemischten und resynthetisierten natürlichen Klängen mit frequenzabhängig unterschiedlichen Einschwingphasen und Dynamikumfängen beruhen, wobei die Lautheit jedes Testsignales gemäß EBU-Empfehlung R128 auf den gleichen Wert gepegelt ist.

Die EBU-Empfehlung R128 (gemäß Fassung aus dem Jahr 2014), "Loudness Normalisation and Permitted Maximum Level of Audio Signals" der Europäischen Rundfunkunion, einem Zusammenschluss von derzeit 73 Rundfunkanstalten in 56 Staaten Europas, Nordafrikas und Vorderasiens, hat quasi Normcharakter und definiert Wege zur Pegelung eines Tonsignals auf Basis des derzeit aktuellsten Modell des psychoakustischen Lautheitsempfindens des Menschen. Sie ist z.B. unter https://tech.ebu.ch/docs/r/r128-2014.pdf abrufbar.

Bei einer bevorzugten Ausführungsform sind die Testsignale Teilfrequenzbänder eines aus natürlichen Klängen mit frequenzabhängig unterschiedlichen Einschwingphasen und Dynamikumfängen zusammengemischten und mit Hilfe eines Envelope Followers und rosa Rauschens resynthetisierten Gesamtklangs. Zweckmäßigerweise lehnt sich die Unterteilung des Gesamtklangs in Teilfrequenzbänder an die Frequenzbänder an, die das zu konfigurierende Hörgerät unterscheidet. Bevorzugt umfasst jedes Testsignal eine Bandbreite von etwa einer halben Oktave bis etwa eineinhalb Oktaven, vorzugsweise eine Bandbreite von etwa einer zweidrittel Oktave bis einer Oktave, wobei die Flankensteilheit jedes Testsignals etwa 92 bis 100 dB/Oktave, vorzugsweise etwa 96 dB/Oktave betragen kann.

Bei einer weiteren bevorzugten Ausführungsform werden sechs bis vierzehn, vorzugsweise neun bis zwölf Testsignale verwendet werden, die jeweils eine Bandbreite von einer Oktave aufweise und vorzugsweise zumindest bestimmte der folgenden Frequenzbänder umfassen: 94 Hz bis 188 Hz, 188 Hz bis 375 Hz, 375 Hz bis 750 Hz, 750 Hz bis 1500 Hz, 1500 Hz bis 3000 Hz, 2250 Hz bis 4500 Hz, 3000 Hz bis 6000 Hz, 3750 Hz bis 7500 Hz, 4500 Hz bis 9000 Hz, 5250 Hz bis 10500 Hz, 6000 Hz bis 12000 Hz, 6750 Hz bis 13500 Hz.

Die genannten natürlichen Klänge können unter anderem insbesondere Sprache, perkussive Klänge und Klänge von Streichinstrumenten mit reichem Obertonspektrum umfassen.

Erfindungsgemäß erfolgt die Konfiguration eines Hörgeräts auf Grundlage von Rückmeldungen des Patienten, da diese das reale Hörempfinden des Trägers genauer widerspiegeln als objektive Messmethoden, wie z.B. die sog. In-situ-Messung, bei der die tatsächlich am Trommelfell anliegende vom Hörgerät erzeugte frequenzabhängige Verstärkung gemessen wird, oder standardisierte Anpassformeln, wie z.B. NAL, DSL, etc.

Das erfindungsgemäße Verfahren stellt sicher, dass alle vom Hörgerät verstärkten Frequenzbereiche als gleichlaut wahrgenommen werden, kompensiert etwaige klangverzerrende Effekte des Hörgerätes und ermöglicht es, einen natürlichen Höreindruck zu erreichen. Vorteilhaft wird bei der erfindungsgemäßen Konfiguration zunächst leisen Klängen im Bereich von etwa 36 dB Aufmerksamkeit geschenkt, um diese über die Hörschwelle des Patienten zu verstärken und zu gewährleisten, dass der Patient auch leise Sprachanteile mühelos wahrnehmen und ähnlich klingende Wörter problemlos differenzieren kann.

Dabei verwendet das erfindungsgemäße Verfahren Testsignale, die verschiedene Frequenzbereiche umfassen und als psychoakustisch gleichlaut empfunden werden, um während der Konfiguration mit diesen Signalen testen zu können, ob die frequenzabhängige Ausgangslautstärke des Hörgerätes ebenfalls als gleichlaut empfunden wird.

Wie laut ein normalhörender Mensch ein Tonsignal empfindet, hängt zu einem großen Teil von der spektralen Verteilung der Teiltöne in dem Signal und ihrer Dichte bzw. ihrer Dynamik (Lautstärkeverlauf oder auch Lautstärkehüllkurve genannt) ab. Ist z.B. der Bassanteil eines Klangs im Verhältnis zu seinem Anteil an hohen Frequenzen sehr hoch, wird der Klang i.d.R. sogar dann als leiser im Vergleich zu einem Klang, der einen ausgewogenen Frequenzverlauf hat, empfunden, wenn letzterer de facto denselben oder sogar einen niedrigeren Lautstärkepegel in dB/Peak aufweist. Des Weiteren weisen die Teilfrequenzen der einzelnen spektralen Bereiche eines natürlichen Klangs (tief, mittel, hoch) unterschiedlich schnelle Einschwingphasen auf. Die hohen Frequenzanteile eines natürlichen Klangs erreichen typischerweise ihren Maximalpegel schneller als die tiefen Frequenzanteile, was ebenfalls Einfluss auf die empfundene Lautheit des Gesamtklangs hat. Aus diesen Gründen gibt es verschiedene psychoakustische Messverfahren, die versuchen, der menschlichen Wahrnehmung von Lautheit so nah wie möglich zu kommen. Im Speziellen sei hier die EBU-Empfehlung R128 genannt, deren Wert "Integrated Loudness" das momentan aktuellste Modell des psychoakustischen Lautheitsempfindens des Menschen darstellt.

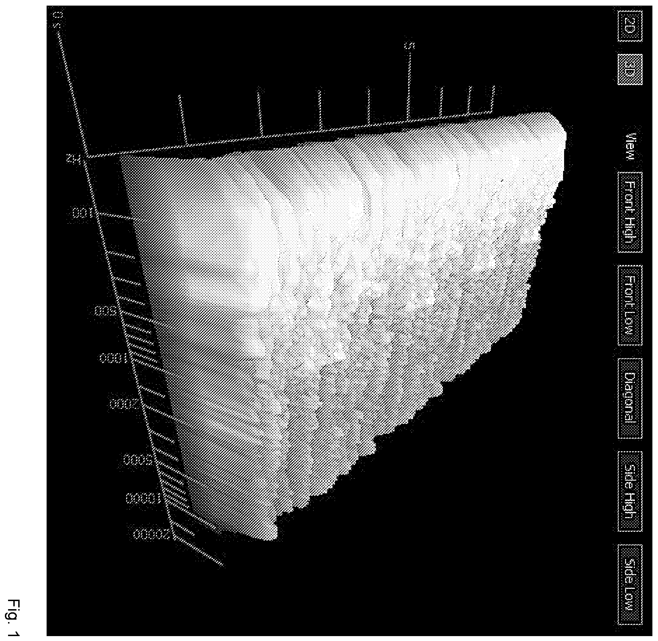

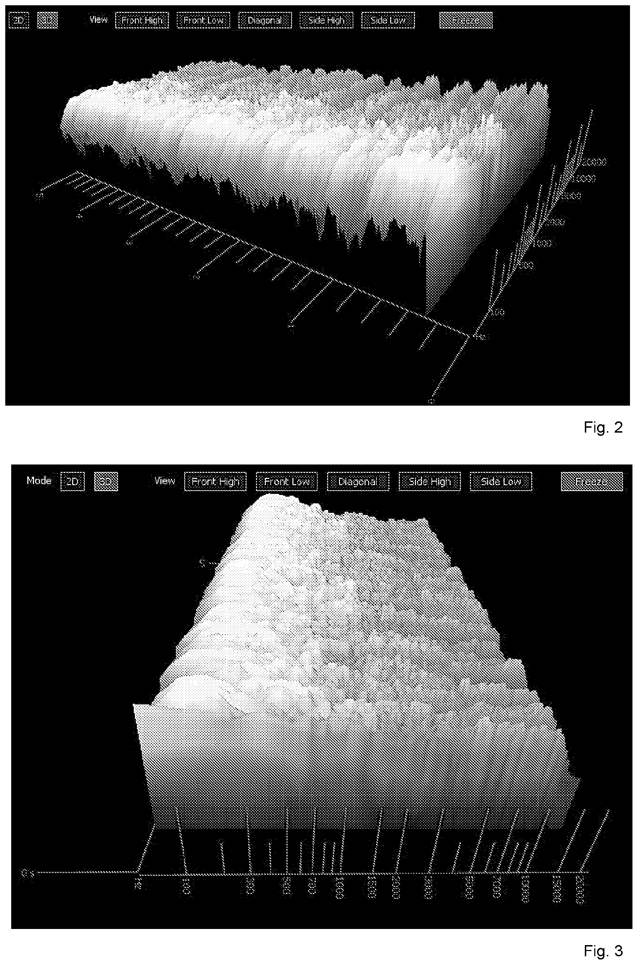

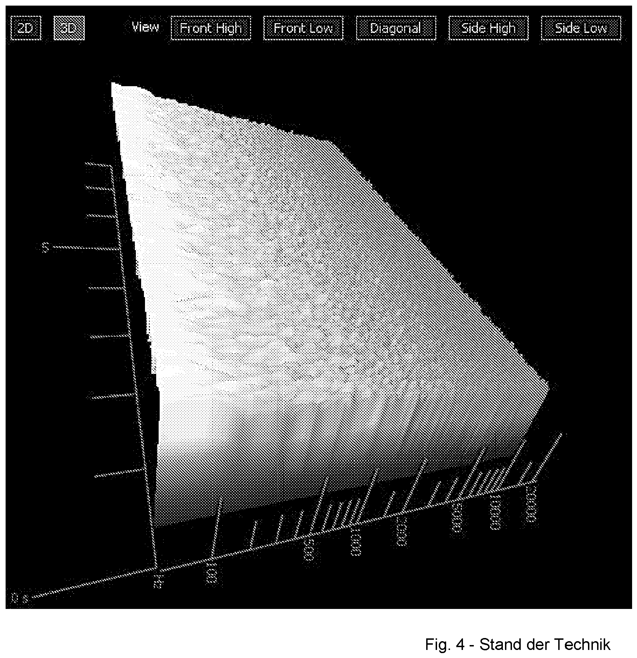

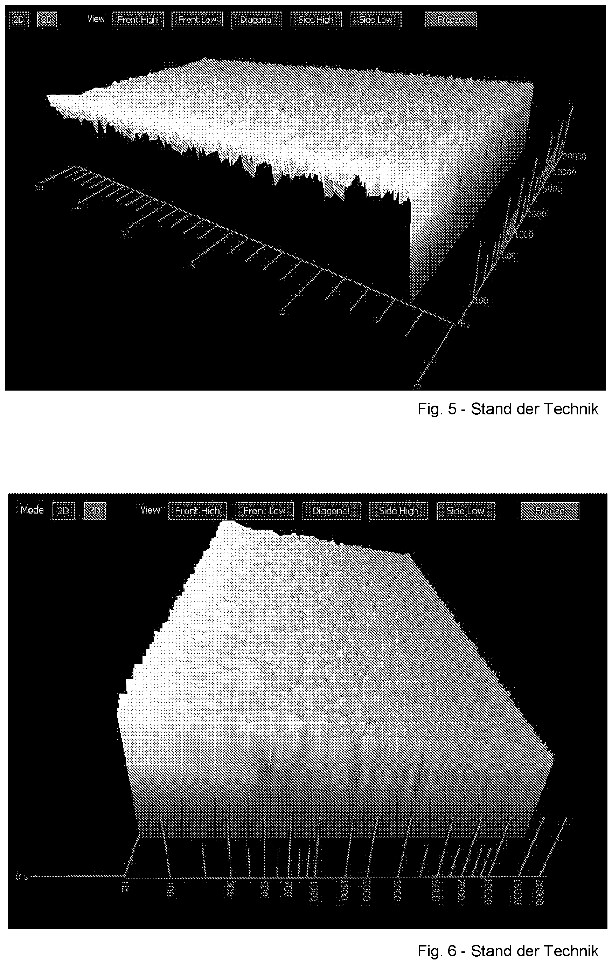

Erfindungsgemäß wird erstmals der psychoakustischen Realität der menschlichen Hörverarbeitung Rechnung getragen, wozu vorzugsweise zunächst natürliche Klänge, die frequenzabhängig unterschiedlich schnelle Einschwingphasen und Dynamikumfänge (Lautstärkehüllkurven) aufweisen, zusammengemischt und mit Hilfe eines Envelope Followers und rosa Rauschens resynthetisiert werden, so dass die Teiltondichte zunimmt, gleichzeitig aber die einzelnen Frequenzbereiche ihr typisches Einschwingverhalten und ihre Dynamik, welche über die jeweils empfundene Lautheit mitentscheiden, beibehalten. Die Figuren 1 bis 3 zeigen ein Spektrogramm eines solchermaßen gewonnen Gesamtklangs aus verschiedenen Perspektiven, während die Figuren 4 bis 6 zum Vergleich ein Spektrogramm eines rosa Rauschens, wie es im Stand der Technik als Testsignal verwendet wird, ebenfalls aus verschiedenen Perspektiven zeigen. Dabei sind in den Figuren 1 bis 6 in der üblichen Weise auf der X-Achse die Frequenz und auf Y-Achse die Zeit aufgetragen, während die Höhe und Helligkeit der Grauschattierung abstrakt den Lautstärkepegel ausdrücken. Je heller und höher ein Punkt ist, desto mehr Schalldruck repräsentiert er.

Die Figuren 1 bis 3 zeigen, dass das Signal im Pegel stark moduliert ist, wobei die Modulation in verschiedenen Frequenzbereichen unterschiedlich ist. Das Signal weist in den höheren Frequenzbereichen eine höhere Dynamik (Unterschied zwischen leisestem und lautestem Pegel) auf als in den tieferen Frequenzbereichen. Zu erkennen ist auch, dass das Signal in den höheren Frequenzbereichen schneller abklingt als in den tieferen Frequenzbereichen. Daher sind mehr "Lücken" in den Höhen zu sehen als in den Tiefen. Die Hüllkurve der hohen Frequenzen unterscheidet sich also stark von der der tiefen Frequenzen. Im Vergleich zum rosa Rauschen (Figuren 4 bis 6) weisen die hohen Frequenzen auch höhere Pegelspitzen, also einen höheren Ausschlag auf.

Das in den Figuren 4 bis 6 zum Vergleich gezeigte rosa Rauschen unterscheidet sich zwischen 125 Hz und 8 kHz um ca. 3 dB/IL zu den hohen Frequenzen hin, mit einem Anstieg um über 4 dB/IL im mittleren Bereich des Frequenzspektrums, der Isophonenkurve folgend, was dazu führt, das Testsignale hoher Frequenzen vom Patienten regelmäßiger als lauter wahrgenommen werden als gleichlaute Testsignale niedrigerer Frequenzen mit der Folge, dass Hörgeräte gemäß den Verfahren des Standes der Technik suboptimal konfiguriert wurden.

Der Gesamtklang wird dann in z.B. zwölf schmalbandige Testsignale unterteilt, die jeweils eine Bandbreite von z.B. zweidrittel einer Oktave oder einer ganze Oktave umfassen bei einer Flankensteilheit von vorzugsweise 96 dB/Oktave. Jedes dieser Testsignale wird nach dem Wert der Integrated Loudness der EBU-Empfehlung R128 proportional auf den gleichen Wert gepegelt, um somit ein moduliertes Rauschen zu erhalten, das über frequenzabhängige natürliche Lautstärkehüllkurven verfügt und über seinen gesamten Frequenzverlauf hinweg vom Normalhörenden als gleichlaut empfunden wird. Die Testsignale werden dann dem Patienten zunächst mit einer niedrigen Lautstärke von z.B. 38 dBA (RMS) und anschließend mit höhere Lautstärke von z.B. 63 dBA (RMS) vorgespielt, um zu überprüfen, ob die eingestellte frequenzabhängige Verstärkung zu einem gleichmäßig lauten Hörempfinden über das gesamte Wiedergabespektrum des Hörgerätes führt. Der Test mit 38 dBA dient dabei insbesondere dazu, sicherzustellen, dass die wortdifferenzierenden Phoneme wie "s" und "t" deutlich wahrgenommen werden. Der Test mit 63 dBA dient dazu, zu überprüfen, ob der Hörgeräteträger ein normallautes Hörempfinden mit dem Hörgerät hat und ob alle klangverzerrenden Effekte der Hörgerätekomponenten und der akustischen Ankopplung erfolgreich ausgeglichen wurden.

Ein typischer Ablauf bei der Durchführung eines erfindungsgemäßen Verfahrens zur Konfiguration zweier Hörgeräte für einen Patienten mit einer Schallempfindungsschwerhörigkeit im Hochtonbereich ist wie folgt, wobei davon ausgegangen wird, dass im Vorfeld eine übliche Tonaudiometrie zur Bestimmung der Schwerhörigkeit durchgeführt wurde.

Zu Beginn der Konfigurationssitzung werden dem Patienten alle z.B. zwölf Testsignale bei 38 dBA Pegel der Reihe nach ohne Hörgeräte vorgespielt, um ihm die Testsignale vorzustellen und gleichzeitig zu überprüfen, bis zu welcher Frequenz er mit seinem natürlichen Hörvermögen leise Pegel wahrnehmen kann. Typischerweise wird der Patient Testsignale über 2 kHz nicht mehr hören. Das Testsignal, welches er dabei noch deutlich wahrnehmen kann, dient im Folgenden als Referenzsignal.

Nun werden ihm die Hörgeräte angelegt oder eingesetzt und in eine Grundeinstellung gebracht, was typischerweise automatisch anhand einer der herkömmlichen Anpassformeln erfolgen kann. Anschließend werden sämtliche adaptiven Funktionen des Hörgerätes (Expander, Geräuschunterdrückung, Sprachhervorhebung, etc.) so weit wie möglich deaktiviert. Abschließend werden nun in der Verstärkungsmatrix einer hörgerätespezifischen Anpasssoftware die obere und mittlere Dynamikstufe der Hörgeräte auf Verstärkungswerte nahe 0 dB gebracht. Die Verstärkungswerte der unteren Dynamikstufe verbleiben in der Grundeinstellung.

Dem Patienten wird nun das Referenzsignal erneut vorgespielt und nachgefragt, ob er das Signal klar und deutlich, wenngleich leise, vernehmen kann. Ist dies der Fall, werden ihm abwechselnd das Referenzsignal und weiteres Testsignal vorgespielt, die der Patient hinsichtlich der von ihm wahrgenommen Lautstärke vergleichen soll. Bevorzugt liegt die Bandbreite des weiteren Testsignals über der Bandbreite des Referenzsignals. Hat das Referenzsignal z.B. eine Bandbreite von 375 Hz bis 750 Hz, bietet sich als weiteres Testsignal eines mit einer Bandbreite von z.B. 1500 Hz bis 3000 Hz an. Falls der Patient die Signale nicht als gleichlaut empfindet, wird der entsprechende frequenzbandspezifische Verstärkungsfaktor geändert, bis das Testsignal als gleichlaut mit dem Referenzsignal empfunden wird. Diese Vorgehensweise wird dann mit einem weiteren Testsignal wiederholt, dessen Bandbreite über der des zweiten Testsignals liegt, also z.B. von 3000 Hz bis 6000 Hz reicht. Anschließend wird mit den Testsignalen fortgefahren, deren Bandbreiten zwischen den bereits gemessenen liegen, also z.B. 750 Hz bis 1500 Hz, 2250 Hz bis 4500 Hz, etc. bis alle Verstärkungsfaktoren der unteren Dynamikstufe so eingestellt sind, dass der Patient die Testsignale als gleichlaut empfindet.

Als nächstes erhöht man den Testpegel auf ca. 63 dBA. Man spielt dem Patienten erneut das Referenzsignal vor und fragt, ob er diese in angenehmer Lautstärke hört. Falls dies nicht der Fall sein sollte, erhöht oder verringert man die Verstärkung der mittleren Dynamikstufe des betreffenden Frequenzbandes in der Verstärkungsmatrix entsprechend. Anschließend verfährt man nach der gleichen Methode wie in den Schritten zuvor, um alle anderen Testsignale dem Referenzsignal in der Lautheit anzugleichen. Die obere Dynamikstufe der Verstärkungsmatrix bleibt dabei unangetastet.

Abschließend reaktiviert man die adaptiven Funktionen des Hörgeräts, soweit vorhanden, und schließt die Konfiguration damit ab. Je nach Art der Hörbeeinträchtigung kann die Konfiguration geringfügig anders als vorstehend beispielhaft beschrieben erfolgen. Es hat sich jedoch besonders bewährt, immer zunächst ein Referenzsignal zu bestimmen, mit dem dann die anderen Testsignale verglichen werden. Im Falle eines Schallleitungsschadens verändert man alle drei Dynamikstufen parallel, so dass keine Kompression zwischen den Dynamikstufen entsteht, sondern nur der Frequenzverlauf des Hörgerätes auf die Verstärkungsbedürfnisse des Patienten abgestimmt wird.

Die Erfindung erlaubt es durch Verwendung psychoakustisch gleichlaut gepegelter dynamischer Testsignale erstmals, eine realistische frequenzabhängige Beurteilung des Augangspegels eines Hörgeräts durch Patienten selbst vornehmen zu lassen. Des Weiteren ist der paradigmatische Ansatz, unterschwellige Signale (Laute wie s und t) über die Hörschwelle hinaus zu verstärken, neu, da in der Fachwelt bislang die Annahme galt, durch eine solche Verstärkung würden die alltäglichen Störgeräusche so stark hervorgehoben, dass sie unangenehmer Lärm in den Vordergrund der auditiven Wahrnehmung rückten. Tatsächlich besitzen als Lärm empfundene Tonsignale wie Straßenlärm, gleichzeitig sprechende Menschen, gegeneinander schlagende Gegenstände aus Metall oder Glas typischerweise Pegel über 50 dBA und werden somit typischerweise von der mittleren Dynamikstufe und nicht von der unteren Dynamikstufe des Hörgeräts verstärkt. Konsonanten und leise Geräusche, wie das Rascheln von Kleidung werden hingegen von der unteren Dynamikstufe des Hörgeräts verstärkt, im Falle des erfindungsgemäßen Verfahrens über die Hörschwelle hinaus, jedoch nicht so sehr, dass sie als laut empfunden würden. Zudem hat sich gezeigt, dass das Gehirn eines Patienten diese leisen Geräusche, nachdem sie nun wieder wahrgenommen werden, nach kurzer Eingewöhnungszeit wieder als unwichtig bewertet und aus der bewusst wahrgenommenen akustischen Umgebung herausfiltert. Um wirklichen Lärm nicht mehr als unangenehm laut erscheinen zu lassen, müssen daher primär die mittlere und die obere Dynamikstufe des Hörgeräts in ihrer Verstärkung, im Vergleich zu den bekannten NAL und DSL-Zielvorgaben, reduziert werden.

Ein großer Vorteil der Erfindung gegenüber der In-situ-Methode ist, dass sie sich keiner standardisierten Anpassformeln als Grundlage der Konfiguration (NAL etc.) bedient, sondern die Verstärkung der unterschiedlichen Frequenz- und Dynamikbereiche auf die Bedürfnisse des Patienten individualisiert, indem sie das psychoakustische Lautheitsempfinden des jeweiligen Patienten in die Konfiguration mit einbezieht, was durch die neuartigen psychoakustisch gleichlaut empfundenen Testsignale möglich wird. Klangverzerrende Variablen eines Hörgerätes können optimal ausgeglichen werden, so dass das Hörgerät natürlicher klingt. Die Gefahr von Messfehlern, wie sie etwa bei der In-situ-Messung auftreten können, entfällt beim erfindungsgemäßen Verfahren gänzlich. Durch den neuartigen Denkansatz, vor allem leise Pegel über die Hörschwelle hinaus zu verstärken, wird dem Patienten ein wesentlich detaillierteres und vollständigeres Klangbild der akustischen Realität dargeboten, als dies mit den herkömmlichen Methoden der Fall ist. Auch die tatsächlichen Lautstärkeverhältnisse realer akustischer Phänomene werden durch das neue Konfigurationsverfahren realistischer abgebildet als bisher. Es hat sich auch gezeigt, dass das erfindungsgemäße Verfahren die Spontanakzeptanz eines Hörgerätes bei den Patienten deutlich erhöht, wodurch mehrmalige, zeitaufwendige Nachkontrollen der Erstkonfiguration vorteilhaft entfallen.

## Patentansprüche

1. Verfahren zum patientenspezifischen Konfigurieren eines Hörgeräts, das eingehende Tonsignale in eine Anzahl verschiedener Frequenzbänder unterteilt und eingangslautstärkenabhängig verstärkt, wobei pro Frequenzband wenigstens drei Verstärkungsfaktoren individuell konfigurierbar sind, nämlich ein erster Verstärkungsfaktor für niedrige Eingangslaustärkepegel, ein zweiter Verstärkungsfaktor für mittlere Eingangslaustärkepegel und ein dritter Verstärkungsfaktor für hohe Eingangslaustärkepegel, und wobei einem das Hörgerät tragenden Patienten Testsignale, die jeweils ein anderes Frequenzband umfassen, mit demselben Eingangslautstärkepegel vorgespielt werden und der Patient angibt, ob er die Testsignale gleichlaut hört,
**dadurch gekennzeichnet, dass**
die Testsignale auf zusammengemischten und resynthetisierten natürlichen Klängen mit frequenzabhängig unterschiedlichen Einschwingphasen und Dynamikumfängen beruhen, wobei die Lautheit jedes Testsignales gemäß EBU-Empfehlung R128 auf den gleichen Wert gepegelt ist.

2. Verfahren nach Anspruch 1, **dadurch gekennzeichnet, dass** die Testsignale Teilfrequenzbänder eines aus natürlichen Klängen mit frequenzabhängig unterschiedlichen Einschwingphasen und Dynamikumfängen zusammengemischten und mit Hilfe eines Envelope Followers und rosa Rauschens resynthetisierten Gesamtklangs sind.

3. Verfahren nach Anspruch 2, **dadurch gekennzeichnet, dass** jedes Testsignal eine Bandbreite von etwa einer halben Oktave bis etwa eineinhalb Oktaven, vorzugsweise eine Bandbreite von etwa einer zweidrittel Oktave bis einer Oktave umfasst.

4. Verfahren nach Anspruch 3, **dadurch gekennzeichnet, dass** jedes Testsignal eine Flankensteilheit von etwa 92 bis 100 dB/Oktave, vorzugsweise etwa 96 dB/Oktave aufweist.

5. Verfahren nach einem der Ansprüche 1 bis 4, **dadurch gekennzeichnet, dass** sechs bis vierzehn, vorzugsweise neun bis zwölf Testsignale verwendet werden, die jeweils eine Bandbreite von einer Oktave aufweisen.

6. Verfahren nach Anspruch 5, **dadurch gekennzeichnet, dass** Testsignale verwendet werden, die zumindest bestimmte der folgenden Frequenzbänder umfassen: 94 Hz bis 188 Hz, 188 Hz bis 375 Hz, 375 Hz bis 750 Hz, 750 Hz bis 1500 Hz, 1500 Hz bis 3000 Hz, 2250 Hz bis 4500 Hz, 3000 Hz bis 6000 Hz, 3750 Hz bis 7500 Hz, 4500 Hz bis 9000 Hz, 5250 Hz bis 10500 Hz, 6000 Hz bis 12000 Hz, 6750 Hz bis 13500 Hz.

7. Verfahren nach einem der Ansprüche 1 bis 6, **dadurch gekennzeichnet, dass** die natürlichen Klänge unter anderem Sprache, perkussive Klänge und Klänge von Streichinstrumenten mit reichem Obertonspektrum umfassen.

8. Verfahren nach einem der Ansprüche 1 bis 7, **dadurch gekennzeichnet, dass** die Testsignale dem Patienten zunächst mit einem Eingangslautstärkepegel im Bereich von etwa 35 bis 42 dB, vorzugsweise 37 bis 39 dB, insbesondere 38 dB vorgespielt werden, um die ersten Verstärkungsfaktoren zu konfigurieren.

9. Verfahren nach Anspruch 8, **dadurch gekennzeichnet, dass** dem Patienten am Beginn der Konfiguration verschiedene, vorzugsweise alle Testsignale vorgespielt und, sofern vorhanden, dasjenige Testsignal als Referenzsignal ausgewählt wird, das er mit seinem natürlichen Hörvermögen noch wahrnimmt, wobei, wenn er mehrere Testsignale noch wahrnehmen kann, dasjenige als Referenzsignal ausgewählt wird, dass das niedrigste Frequenzband umfasst, und dass dem Patienten nach Auswahl eines Referenzsignals jeweils abwechselnd das Referenzsignal und ein anderes Testsignal vorgespielt werden und der jeweilige erste Verstärkungsfaktor am Hörgerät so eingestellt wird, dass der Patient beide Signale als gleichlaut empfindet.

10. Verfahren nach einem der Ansprüche 8 oder 9, **dadurch gekennzeichnet, dass** nach Abschluss der Konfiguration der ersten Verstärkungsfaktoren dem Patienten die Testsignale mit einem Eingangslautstärkepegel im Bereich von etwa 60 bis 66 dB, vorzugsweise 62 bis 64 dB, insbesondere 63 dB vorgespielt werden, um die zweiten Verstärkungsfaktoren zu konfigurieren.

11. Verfahren zum Erzeugen von Testsignalen zum patientenspezifischen Konfigurieren eines Hörgeräts, wobei die Testsignale jeweils ein anderes Frequenzband umfassen, **gekennzeichnet durch**
- Zusammenmischen verschiedener natürlicher Klängen aus verschiedenen Frequenzbereichen mit frequenzabhängig unterschiedlichen Einschwingphasen und Dynamikumfängen,
- Resynthetisieren der Klänge mit Hilfe eines Envelope Followers und rosa Rauschens derart, dass die Teiltondichte zunimmt und die einzelnen Frequenzbereiche ihr typisches Einschwingverhalten und ihre Dynamik beibehalten,
- Aufteilen des so gewonnen Gesamtklangs in Testsignale unterschiedlicher Frequenzbänder,
- Pegeln jedes Testsignals auf denselben Wert der Lautheit gemäß EBU-Empfehlung R128.

12. Verfahren nach Anspruch 11, **dadurch gekennzeichnet, dass** der Gesamtklang in Testsignale aufgeteilt wird, die zumindest bestimmte der folgenden Frequenzbänder umfassen: 94 Hz bis 188 Hz, 188 Hz bis 375 Hz, 375 Hz bis 750 Hz, 750 Hz bis 1500 Hz, 1500 Hz bis 3000 Hz, 2250 Hz bis 4500 Hz, 3000 Hz bis 6000 Hz, 3750 Hz bis 7500 Hz, 4500 Hz bis 9000 Hz, 5250 Hz bis 10500 Hz, 6000 Hz bis 12000 Hz, 6750 Hz bis 13500 Hz.

13. Verwendung eines gemäß einem der Ansprüche 11 oder 12 erzeugten Testsignals zur patientenspezifischen Konfiguration eines Hörgeräts.

## Claims

1. A method for patient-specific configuration of a hearing aid which divides incoming audio signals into a number of different frequency bands and amplifies them in an input volume-dependent manner, wherein at least three amplification factors are individually configurable per frequency band, namely a first amplification factor for low input volume levels, a second amplification factor for medium input volume levels and a third amplification factor for high input volume levels, and wherein test signals, each comprising a different frequency band, are played to a patient wearing the hearing aid at the same input volume level and the patient indicates whether he hears the test signals as being of equal volume,
**characterized in that**
the test signals are based on mixed and resynthesised natural sounds with frequency-dependent different transient phases and dynamic ranges, whereby the loudness of each test signal is levelled to the same value according to EBU Recommendation R128.

2. The method according to claim 1, **characterized in that** the test signals are partial frequency bands of an overall sound which is mixed together from natural sounds with frequency-dependent different transient phases and dynamic ranges and is resynthesized with the aid of an envelope follower and pink noise.

3. The method according to claim 2, **characterized in that** each test signal comprises a bandwidth of about half an octave to about one and a half octaves, preferably a bandwidth of about two thirds of an octave to one octave.

4. The method according to claim 3, **characterized in that** each test signal has an edge steepness of about 92 to 100 dB/octave, preferably about 96 dB/octave.

5. The method according to any one of claims 1 to 4, **characterized in that** six to fourteen, preferably nine to twelve test signals are used, each having a bandwidth of one octave.

6. The method according to claim 5, **characterized in that** test signals are used which comprise at least certain of the following frequency bands: 94 Hz to 188 Hz, 188 Hz to 375 Hz, 375 Hz to 750 Hz, 750 Hz to 1500 Hz, 1500 Hz to 3000 Hz, 2250 Hz to 4500 Hz, 3000 Hz to 6000 Hz, 3750 Hz to 7500 Hz, 4500 Hz to 9000 Hz, 5250 Hz to 10500 Hz, 6000 Hz to 12000 Hz, 6750 Hz to 13500 Hz.

7. The method according to any one of claims 1 to 6, **characterized in that** the natural sounds include, but are not limited to, speech, percussive sounds, and sounds of stringed instruments having a rich overtone spectrum.

8. The method according to any one of claims 1 to 7, **characterized in that** the test signals are first played to the patient at an input volume level in the range of about 35 to 42 dB, preferably 37 to 39 dB, more preferably 38 dB, to configure the first gain factors.

9. The method according to claim 8, **characterized in that** at the beginning of the configuration different, preferably all, test signals are played to the patient and, if present, that test signal is selected as reference signal which he can still perceive with his natural hearing, wherein, if he can still perceive several test signals, the one which comprises the lowest frequency band is selected as reference signal, and **in that**, after selection of a reference signal, the reference signal and another test signal are alternately played back to the patient and the respective first amplification factor is set on the hearing aid in such a way that the patient perceives both signals as being equally loud.

10. The method according to any one of claims 8 or 9, **characterized in that** after the configuration of the first gain factors is completed, the test signals are played to the patient at an input volume level in the range of about 60 to 66 dB, preferably 62 to 64 dB, more preferably 63 dB, to configure the second gain factors.

11. A method of generating test signals for patient-specific configuration of a hearing aid, the test signals each comprising a different frequency band, **characterized by**
- mixing of different natural sounds from different frequency ranges with frequency-dependent different transient phases and dynamic ranges,
- resynthesizing the sounds with the help of an envelope follower and pink noise in such a way that the partial tone density increases and the individual frequency ranges retain their typical transient response and dynamics,
- splitting the total sound obtained in this way into test signals of different frequency bands,
levelling of each test signal to the same value of loudness according to EBU Recommendation R128.

12. The method according to claim 11, **characterized in that** the total sound is divided into test signals comprising at least certain of the following frequency bands: 94 Hz to 188 Hz, 188 Hz to 375 Hz, 375 Hz to 750 Hz, 750 Hz to 1500 Hz, 1500 Hz to 3000 Hz, 2250 Hz to 4500 Hz, 3000 Hz to 6000 Hz, 3750 Hz to 7500 Hz, 4500 Hz to 9000 Hz, 5250 Hz to 10500 Hz, 6000 Hz to 12000 Hz, 6750 Hz to 13500 Hz.

13. Use of a test signal generated according to any one of claims 11 or 12 for patient-specific configuration of a hearing aid.

## Revendications

1. Procédé de configuration spécifique au patient d'une prothèse auditive qui divise les signaux sonores entrants en un nombre de bandes de fréquences différentes et amplifie ceux-ci en fonction du volume d'entrée, dans lequel au moins trois facteurs de gain sont configurables individuellement par bande de fréquences, à savoir un premier facteur de gain pour des niveaux de volume d'entrée faibles, un deuxième facteur de gain pour des niveaux de volume d'entrée moyens et un troisième facteur de gain pour des niveaux de volume d'entrée élevés, et dans lequel on fait écouter, au même niveau de volume d'entrée, des signaux de test qui comprennent chacun une bande de fréquence différente, à un patient portant la prothèse auditive, et le patient indique s'il entend les signaux de test comme ayant le même volume,
**caractérisé en ce que**
les signaux de test sont basés sur des sons naturels mélangés et resynthétisés avec différentes phases transitoires et plages dynamiques dépendant de la fréquence, dans lequel l'intensité sonore de chaque signal de test est nivelée à la même valeur conformément à la recommandation EBU R128.

2. Procédé selon la revendication 1, **caractérisé par le fait que** les signaux de test sont des bandes de fréquences partielles d'un son global mélangé à partir de sons naturels avec différentes phases transitoires et plages dynamiques dépendant de la fréquence et resynthétisé à l'aide d'un suiveur d'enveloppe et de bruit rose.

3. Procédé selon la revendication 2, **caractérisé en ce que** chaque signal de test comprend une largeur de bande d'environ une demi-octave à environ une octave et demie, de préférence une largeur de bande d'environ deux tiers d'octave à une octave.

4. Procédé selon la revendication 3, **caractérisé en ce que** chaque signal de test présente une pente de front d'environ 92 à 100 dB/octave, de préférence d'environ 96 dB/octave.

5. Procédé selon l'une quelconque des revendications 1 à 4, **caractérisé en ce que** l'on utilise six à quatorze, de préférence neuf à douze signaux de test, qui présentent chacun une largeur de bande d'une octave.

6. Procédé selon la revendication 5, **caractérisé en ce que** l'on utilise des signaux de test qui comprennent au moins certaines des bandes de fréquences suivantes: 94 Hz à 188 Hz, 188 Hz à 375 Hz, 375 Hz à 750 Hz, 750 Hz à 1500 Hz , 1500 Hz à 3000 Hz, 2250 Hz à 4500 Hz, 3000 Hz à 6000 Hz, 3750 Hz à 7500 Hz, 4500 Hz à 9000 Hz, 5250 Hz à 10500 Hz, 6000 Hz à 12000 Hz, 6750 Hz à 13500 Hz.

7. Procédé selon l'une quelconque des revendications 1 à 6, **caractérisé en ce que** les sons naturels comprennent entre autres la parole, les sons de percussion et les sons d'instruments à cordes à spectre harmonique riche.

8. Procédé selon l'une quelconque des revendications 1 à 7, **caractérisé en ce que** l'on fait écouter au patient les signaux de test d'abord à un niveau de volume d'entrée dans la plage d'environ 35 à 42 dB, de préférence de 37 à 39 dB, en particulier de 38 dB, afin de configurer les premiers facteurs de gain.

9. Procédé selon la revendication 8, **caractérisé en ce que**, au début de la configuration, on fait écouter au patient différents signaux de test, de préférence tous les signaux de test, et, s'il est disponible, on choisit le signal de test en tant que signal de référence, qu'il perçoit encore avec son audition naturelle, dans lequel, s'il peut encore percevoir plusieurs signaux de test, on choisit le signal de test en tant que signal de référence, qui comprend la bande de fréquence la plus basse, et que, après avoir choisi un signal de référence, on fait écouter au patient respectivement alternativement le signal de référence et un autre signal de test, et le premier facteur de gain respectif est réglé sur la prothèse auditive de telle sorte que le patient perçoit les deux signaux comme ayant le même volume.

10. Procédé selon l'une quelconque des revendications 8 ou 9, **caractérisé en ce qu'**une fois la configuration des premiers facteurs de gain terminée, on fait écouter au patient les signaux de test à un niveau de volume d'entrée compris entre environ 60 et 66 dB, de préférence entre 62 et 64 dB, en particulier de 63 dB, afin de configurer les deuxièmes facteurs de gain.

11. Procédé de génération de signaux de test pour la configuration spécifique au patient d'une prothèse auditive, dans lequel les signaux de test comprennent chacun une bande de fréquence différente, **caractérisé par**
- mélanger différents sons naturels de différentes plages de fréquences avec différentes phases transitoires et plages dynamiques dépendant de la fréquence,
- resynthétiser les sons à l'aide d'un suiveur d'enveloppe et de bruit rose de manière à ce que la densité de tonalité partielle augmente et que les plages de fréquences individuelles conservent leur comportement transitoire et leur dynamique typiques,
- diviser le son global ainsi obtenu en signaux de test de différentes bandes de fréquences,
- niveler chaque signal de test à la même valeur d'intensité sonore conformément à la recommandation EBU R128.

12. Procédé selon la revendication 11, **caractérisé en ce que** le son global est divisé en signaux de test qui comprennent au moins certaines des bandes de fréquences suivantes: 94 Hz à 188 Hz, 188 Hz à 375 Hz, 375 Hz à 750 Hz, 750 Hz à 1500 Hz , 1500 Hz à 3000 Hz, 2250 Hz à 4500 Hz, 3000 Hz à 6000 Hz, 3750 Hz à 7500 Hz, 4500 Hz à 9000 Hz, 5250 Hz à 10500 Hz, 6000 Hz à 12000 Hz, 6750 Hz à 13500 Hz.

13. Utilisation d'un signal de test généré selon l'une quelconque des revendications 11 ou 12, pour la configuration spécifique au patient d'une prothèse auditive.
